(19) Europäisches Patentamt
European Patent Office
Office européen des brevets

(11) Veröffentlichungsnummer: **0 269 916**
**A1**

(12) # EUROPÄISCHE PATENTANMELDUNG

(21) Anmeldenummer: 87116507.2

(22) Anmeldetag: 09.11.87

(51) Int. Cl.4: C07D 275/06 , C07D 295/08 , C07D 265/30 , C07D 211/14 , A01N 43/80 , A01N 43/40 , A01N 43/84

(30) Priorität: 22.11.86 DE 3639902

(43) Veröffentlichungstag der Anmeldung: 08.06.88 Patentblatt 88/23

(84) Benannte Vertragsstaaten: BE CH DE FR GB IT LI NL

(71) Anmelder: BAYER AG
Konzernverwaltung RP Patentabteilung
D-5090 Leverkusen 1 Bayerwerk(DE)

(72) Erfinder: Krämer, Wolfgang, Dr.
Rosenkranz 25
D-5093 Burscheid 2(DE)
Erfinder: Weissmüller, Joachim, Dr.
Carl-Langhans-Strasse 53
D-4019 Monheim(DE)
Erfinder: Reinecke, Paul, Dr.
Steinstrasse 8
D-5090 Leverkusen 3(DE)
Erfinder: Hänssler, Gerd, Dr.
Am Arenzberg 58a
D-5090 Leverkusen 3(DE)

(54) **Saccharin-Salze von substituierten Hydroxypropylaminen.**

(57) Neue Saccharin-Salze von substituierten Hydroxypropylaminen der allgemeinen Formel (I),

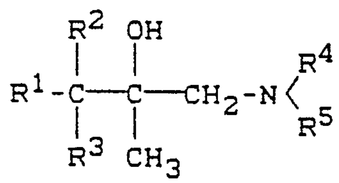

x

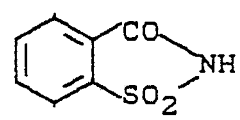

(I)

in welcher
$R^1$ bis $R^5$ die in der Beschreibung gegebenen Bedeutungen haben und ihre Verwendung zur Bekämpfung von Schädlingen.

Die neuen Verbindungen der allgemeinen Formel (I) können aus geeigneten Hydroxypropylaminen und Saccharin hergestellt werden.

EP 0 269 916 A1

BAYER AKTIENGESELLSCHAFT
Konzernverwaltung RP
Patentabteilung

5090 Leverkusen, Bayerwerk

Bas/Ke-c
(Ia)

# Saccharin-Salze von substituierten Hydroxypropylaminen

Die vorliegende Erfindung betrifft neue Saccharin-Salze von substituierten Hydroxypropylaminen, ein Verfahren zu ihrer Herstellung und ihre Verwendung als Schädlingsbekämpfungsmittel.

Es ist bereits bekannt, daß Saccharin-Salze von substituierten Aminen, wie beispielsweise das Saccharin-Salz des 5-Amino-1,2,4-triazols oder das Saccharin-Salz des Cyclohexylamins fungizide Eigenschaften besitzen (vgl. EP 158 074).

Weiterhin ist bekannt, daß bestimmte substituierte Hydroxyalkylamine, wie beispielsweise das 4-(4-t-Butylphenyl)-3-methyl-1-(3-methyl-piperidin-1-yl)-butan-2-ol fungizide Eigenschaften besitzen (vgl. EP 129 321).

Die Wirksamkeit dieser vorbekannten Verbindungen ist jedoch insbesondere bei niedrigen Aufwandmengen und Konzentrationen nicht in allen Anwendungsbereichen völlig zufriedenstellend.

Es wurden neue Saccharin-Salze von substituierten Hydroxypropylaminen der allgemeinen Formel (I),

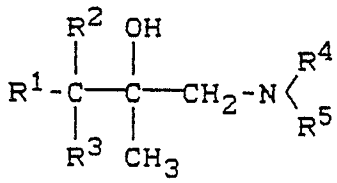 x 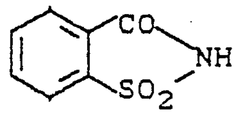 (I)

in welcher

$R^1$ für jeweils gegebenenfalls substituiertes Phenyl, Phenoxy, Phenylthio, Phenylalkyl, Phenoxyalkyl und Phenylthioalkyl oder für jeweils gegebenenfalls substituiertes Cyclohexyl, Cyclohexyloxy, Cyclohexylthio, Cyclohexylalkyl, Cyclohexyloxyalkyl und Cyclohexylthioalkyl steht,

$R^2$ für Wasserstoff oder Methyl steht,

$R^3$ für Methyl oder Ethyl steht und

$R^4$ und $R^5$ unabhängig voneinander für Alkyl oder Alkenyl stehen oder

$R^4$ und $R^5$ gemeinsam mit dem Stickstoffatom, an das sie gebunden sind, für einen gegebenenfalls substituierten, gesättigten Heterocyclus stehen, der gegebenenfalls weitere Heteroatome enthalten kann,

gefunden.

Die Verbindungen der Formel (I) können als geometrische und/oder optische Isomere oder Isomerengemische unterschiedlicher Zusammensetzung anfallen. Sowohl die reinen Isomeren als auch die Isomerengemische werden erfindungsgemäß beansprucht.

Weiterhin wurde gefunden, daß man die neuen Saccharin-Salze von substituierten Hydroxypropylaminen der allgemeinen Formel (I),

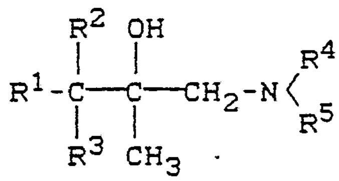 x 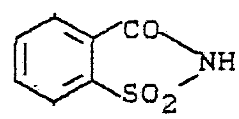 (I)

in welcher

$R^1$ für jeweils gegebenenfalls substituiertes Phenyl, Phenoxy, Phenylthio, Phenylalkyl, Phenoxyalkyl und Phenylthioalkyl oder für jeweils gegebenenfalls substituiertes Cyclohexyl, Cyclohexyloxy, Cyclohexylthio, Cyclohexylalkyl, Cyclohexyloxyalkyl und Cyclohexylthioalkyl steht,

$R^2$ für Wasserstoff oder Methyl steht,

$R^3$ für Methyl oder Ethyl steht und

$R^4$ und $R^5$ unabhängig voneinander für Alkyl oder Alkenyl stehen oder

$R^4$ und $R^5$ gemeinsam mit dem Stickstoffatom, an das sie gebunden sind, für einen gegebenenfalls substituierten, gesättigten Heterocyclus stehen, der gegebenenfalls weitere Heteroatome enthalten kann,

erhält, wenn man substituierte Hydroxypropylamine der Formel (II),

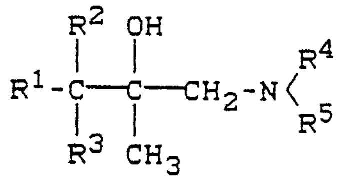

(II)

in welcher

$R^1$, $R^2$, $R^3$, $R^4$ und $R^5$ die oben angegebene Bedeutung haben,

mit Saccharin gegebenenfalls in Gegenwart eines Verdünnungsmittels umsetzt.

Schließlich wurde gefunden, daß die neuen Saccharin-Salze von substituierten Hydroxypropylaminen der allgemeinen Formel (I) eine Wirkung gegen Schädlinge besitzen.

Überraschenderweise zeigen die erfindungsgemäßen Saccharin-Salze von substituierten Hydroxypropylaminen der allgemeinen Formel (I) u.a. eine bessere fungizide Wirksamkeit als die aus dem Stand der Technik bekannten Sac-

charin-Salze von substituierten Aminen, wie beispielsweise das Saccharin-Salz des 5-Amino-1,2,4-triazols oder das Saccharin-Salz des Cyclohexylamins und ebenfalls eine erheblich bessere fungizide Wirksamkeit als die aus dem Stand der Technik bekannten substituierten Hydroxyalkylamine, wie beispielsweise das 4-(4-t-Butylphenyl)-3-methyl-1-(3-methyl-piperidin-1-yl)-butan-2-ol, welche chemisch und wirkungsmäßig naheliegende Verbindungen sind.

Die erfindungsgemäßen Saccharin-Salze von substituierten Hydroxypropylaminen sind durch die Formel (I) allgemein definiert. Bevorzugt sind Verbindungen der Formel (I), bei welchen

$R^1$ für jeweils gegebenenfalls einfach bis fünffach, gleich oder verschieden substituiertes Phenyl, Phenoxy, Phenylthio, Phenylalkyl, Phenoxyalkyl und Phenylthioalkyl mit jeweils 1 oder 2 Kohlenstoffatomen im Alkylteil steht, wobei als Phenylsubstituenten jeweils infrage kommen: Halogen, jeweils geradkettiges oder verzweigtes Alkyl, Alkoxy oder Alkylthio mit jeweils 1 bis 5 Kohlenstoffatomen, Halogenalkyl, Halogenalkoxy oder Halogenalkylthio mit jeweils 1 oder 2 Kohlenstoffatomen und 1 bis 5 gleichen oder verschiedenen Halogenatomen, wie vorzugsweise Fluor- oder Chloratome; oder für jeweils gegebenenfalls einfach oder mehrfach, gleich oder verschieden substituiertes Cyclohexyl, Cyclohexyloxy, Cyclohexylthio, Cyclohexylalkyl, Cyclohexyloxyalkyl und Cyclohexylthioalkyl mit

jeweils 1 oder 2 Kohlenstoffatomen im Alkylteil steht, wobei als Cyclohexylsubstituenten jeweils infrage kommen: jeweils geradkettiges oder verzweigtes Alkyl oder Alkoxy mit jeweils 1 bis 5 Kohlenstoffatomen, Trifluormethyl oder Trifluormethoxy,

$R^2$ für Wasserstoff oder Methyl steht,

$R^3$ für Methyl oder Ethyl steht und

$R^4$ und $R^5$ unabhängig voneinander für geradkettiges oder verzweigtes Alkyl mit 1 bis 8 Kohlenstoffatomen oder für geradkettiges oder verzweigtes Alkenyl mit 3 bis 6 Kohlenstoffatomen stehen oder

$R^4$ und $R^5$ gemeinsam mit dem Stickstoffatom, an welches sie gebunden sind, für einen gegebenenfalls einfach bis zehnfach, gleich oder verschieden substituierten 5- bis 7-gliedrigen, gesättigten Heterocyclus mit 1 oder 2 Heteroatomen, vorzugsweise Stickstoff oder Sauerstoff stehen, wobei als Substituenten infrage kommen: Methyl, Ethyl und Hydroxymethyl.

Besonders bevorzugt sind Verbindungen der allgemeinen Formel (I), bei welchen

$R^1$ für jeweils gegebenenfalls einfach bis dreifach, gleich oder verschieden substituiertes Phenyl,

Phenylmethyl, Phenoxymethyl, Phenylthiomethyl, Phenoxy oder Phenylthio steht, wobei als Phenylsubstituenten jeweils infrage kommen: Fluor, Chlor, Brom, Methyl, Ethyl, n-Propyl, i-Propyl, n-, i-, s- oder t-Butyl, n-Pentyl, 2-Methyl-2-butyl, Methoxy, Ethoxy, n- oder i-Propoxy, t-Butoxy, Trifluormethyl, Trifluormethoxy und Trifluormethylthio; oder für jeweils gegebenenfalls einfach bis dreifach, gleich oder verschieden substituiertes Cyclohexyl, Cyclohexylmethyl, Cyclohexyloxymethyl, Cyclohexylthiomethyl, Cyclohexyloxy oder Cyclohexylthio steht, wobei als Cyclohexylsubstituenten jeweils infrage kommen: Methyl, Ethyl, n- oder i-Propyl, n-, i-, s- oder t-Butyl, n-Pentyl, 2-Methyl-2-butyl, Methoxy, Ethoxy, n- oder i-Propoxy, t-Butoxy, Trifluormethyl und Trifluormethoxy,

$R^2$ für Wasserstoff oder Methyl steht,

$R^3$ für Methyl oder Ethyl steht und

$R^4$ und $R^5$ unabhängig voneinander für Methyl, Ethyl, n- oder i-Propyl, n-, i-, s- oder t-Butyl, n-Pentyl, Allyl, 2-Butenyl oder 3-Methyl-2-butenyl stehen oder

$R^4$ und $R^5$ gemeinsam mit dem Stickstoffatom, an welches sie gebunden sind, für jeweils gegebenenfalls einfach bis dreifach, gleich oder verschieden durch Methyl, Ethyl oder Hydroxymethyl substituiertes 1-Pyrrolidinyl, 1-Piperidinyl, 1-Piperazinyl, 4-Morpholinyl oder 1-Hexahydroazepinyl stehen.

Ganz besonders bevorzugt sind Verbindungen der allgemeinen Formel (I), bei welchen

$R^1$ für jeweils gegebenenfalls ein- oder zweifach, gleich oder verschieden durch Chlor, Methyl, t-Butyl und Trifluormethoxy substituiertes Phenyl, Phenoxy, Phenoxymethyl, Phenylthiomethyl, Phenylthio, Cyclohexyloxy, Cyclohexylmethyl oder Cyclohexyloxymethyl steht,

$R^2$ für Wasserstoff oder Methyl steht,

$R^3$ für Methyl steht und

$R^4$ und $R^5$ gemeinsam mit dem Stickstoffatom, an dem sie stehen, für gegebenenfalls ein- bis dreifach durch Methyl substituiertes 1-Piperidinyl, 4-Morpholinyl oder 1-Hexahydroazepinyl stehen.

Im einzelnen seien außer den bei den Herstellungsbeispielen genannten Verbindungen die folgenden Verbindungen der allgemeinen Formel (I) genannt:

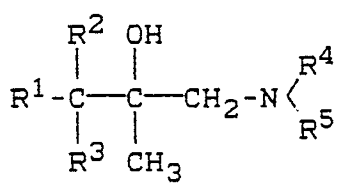

x

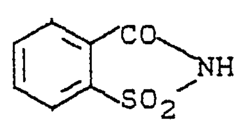

(I)

Tabelle 1

| $R^1$ | $R^2$ | $R^3$ | $-N\langle^{R^4}_{R^5}$ |
|---|---|---|---|
| 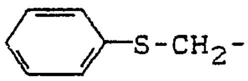 | $CH_3$ | $CH_3$ | 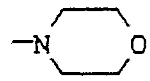 |
| 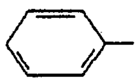 | $CH_3$ | $CH_3$ | 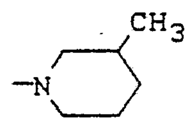 |
| 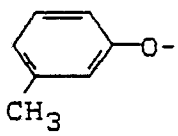 | $CH_3$ | $CH_3$ | 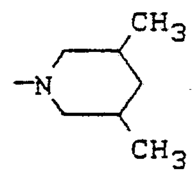 |
| 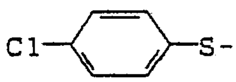 | $CH_3$ | $CH_3$ | 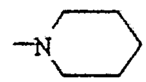 |
| 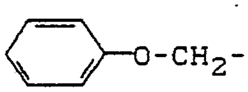 | $CH_3$ | $CH_3$ | 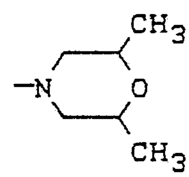 |
| 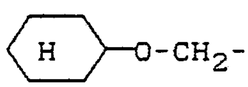 | $CH_3$ | $CH_3$ | 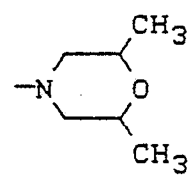 |
| 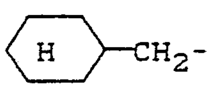 | $CH_3$ | $CH_3$ | 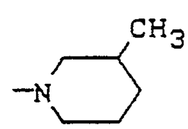 |
| 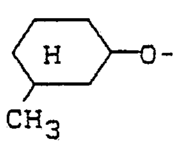 | $CH_3$ | $CH_3$ | 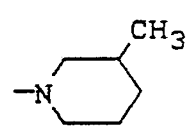 |

Tabelle 1 - Fortsetzung

| $R^1$ | $R^2$ | $R^3$ | $-N\langle^{R^4}_{R^5}$ |
|---|---|---|---|
| 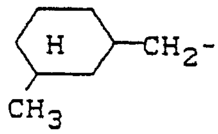 | H | $CH_3$ | 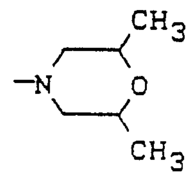 |
| 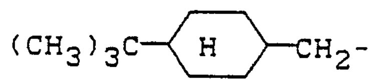 | H | $CH_3$ | 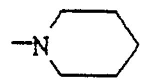 |
| 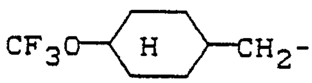 | $CH_3$ | $CH_3$ | 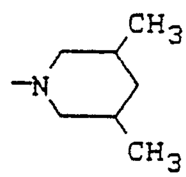 |
| 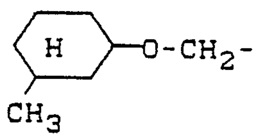 | $CH_3$ | $CH_3$ | 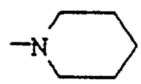 |

Verwendet man beispielsweise 2,3,3-Trimethyl-4-(3-methylphenyl)-1-(morpholin-4-yl)-2-butanol als Ausgangsverbindung, so kann der Reaktionsablauf des erfindungsgemäßen Verfahrens durch das folgende Formelschema dargestellt werden:

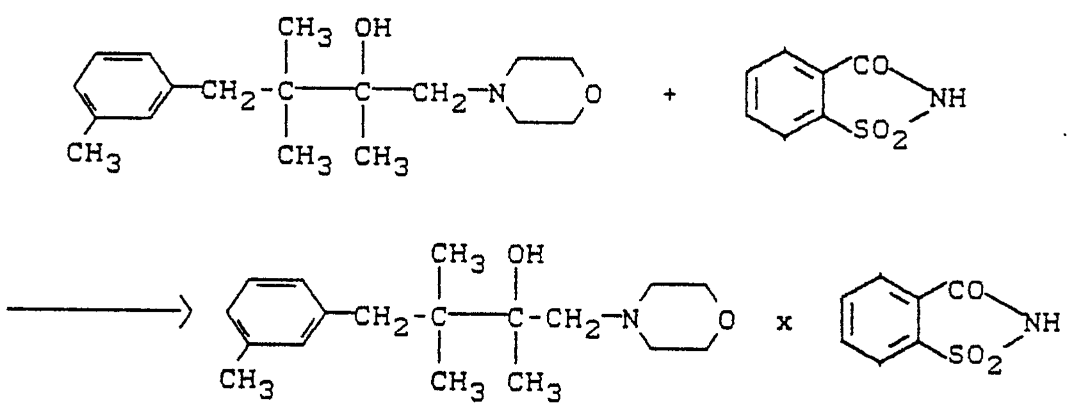

Die zur Durchführung des erfindungsgemäßen Verfahrens als Ausgangsstoffe benötigten substituierten Hydroxypropylamine sind durch die Formel (II) allgemein definiert. In dieser Formel (II) stehen $R^1$, $R^2$, $R^3$, $R^4$ und $R^5$ für diejenigen Reste, die bereits im Zusammenhang mit der Beschreibung der erfindungsgemäßen Stoffe der Formel (I) für diese Substituenten genannt wurden.

Die substituierten Hydroxypropylamine sind teilweise bekannt (vgl. DE-OS 25 05 423). Teilweise sind sie Gegenstand einer eigenen vorgängigen noch nicht publizierten Patentanmeldung (vgl. Deutsche Patentanmeldung P 3 627 071 vom 9. August 1986).

Man erhält sie beispielsweise, wenn man Epoxide der Formel (III),

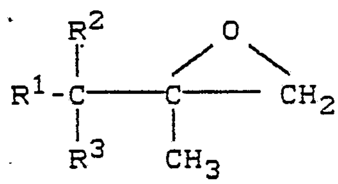

(III)

in welcher

$R^1$, $R^2$ und $R^3$ die oben angegebene Bedeutung haben,

mit Aminen der allgemeinen Formel (IV),

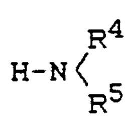

(IV)

in welcher

$R^4$ und $R^5$ die oben angegebene Bedeutung haben,

gegebenenfalls in Gegenwart eines Verdünnungsmittels, wie beispielsweise n-Butanol, und gegebenenfalls in Gegenwart eines Katalysators, wie beispielsweise Essigsäure, bei Temperaturen zwischen 40 °C und 200 °C umsetzt und gegebenenfalls anschließend in einer 2. Stufe die so erhältlichen substituierten Hydroxypropylamine der Formel (IIa),

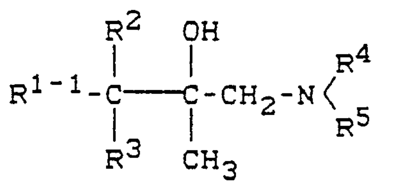
(IIa)

in welcher

$R^{1-1}$ für jeweils gegebenenfalls substituiertes Phenyl, Phenoxy, Phenylalkyl oder Phenoxyalkyl steht und

$R^2$, $R^3$, $R^4$ und $R^5$ die oben angegebene Bedeutung haben,

gegebenenfalls in Gegenwart eines Verdünnungsmittels, wie beispielsweise Isopropanol, und gegebenenfalls in Gegenwart eines Katalysators, wie beispielsweise Ruthenium auf Kohlenstoff, bei Temperaturen zwischen 80 °C und 200 °C in üblicher Art und Weise mit molekularem Wasserstoff im Phenylring hydriert.

Die Epoxide der Formel (III) sind bekannt (vgl. z.B. DE-OS 34 13 996) oder können in allgemein bekannter Art und Weise erhalten werden, beispielsweise indem man Methyl-

ketone der Formel (V),

$$R^1-\overset{\overset{\displaystyle R^2}{|}}{\underset{\underset{\displaystyle R^3}{|}}{C}}-CO-CH_3 \qquad (V)$$

in welcher

$R^1$, $R^2$ und $R^3$ die oben angegebene Bedeutung haben,

entweder

α) mit Dimethyloxosulfonium-methylid der Formel (VI)

$$(CH_3)_2\underset{\underset{\displaystyle O}{\|}}{S}^{\delta+}=CH_2^{\delta-} \qquad (VI)$$

in an sich bekannter Weise in Gegenwart eines Verdünnungsmittels, wie beispielsweise Dimethylsulfoxid, bei Temperaturen zwischen 20 °C und 80 °C umsetzt (vgl. hierzu die Angaben in J.Am.Soc. 87, 1363-1364 [1965]),

oder

β) mit Trimethylsulfonium-methylsulfat der Formel (VII)

$$[(CH_3)_3S^+]\ \ CH_3SO_4^- \qquad (VII)$$

in an sich bekannter Weise in Gegenwart eines inerten organischen Lösungsmittels, wie beispielsweise Acetonitril, und in Gegenwart einer Base, wie beispielsweise Natriummethylat, bei Temperaturen

zwischen 0 °C und 60 °C, vorzugsweise bei Raumtemperatur, umsetzt (vgl. auch die Angaben in Heterocycles 8, 397 [1977]).

Die Methylketone der Formel (V) sind bekannt (vgl. z. B. DE-OS 30 48 266 und DE-OS 32 10 725) oder können nach den dort angegebenen Verfahren in analoger Weise erhalten werden.

Die Epoxide der Formel (III) können auch erhalten werden, wenn man entsprechende Olefine nach prinzipiell bekannten Verfahren beispielsweise durch Umsetzung mit Wasserstoffperoxid oder mit Persäuren epoxidiert.

Die Amine der Formel (IV) sind allgemein bekannte Verbindungen der organischen Chemie.

Als Verdünnungsmittel zur Durchführung des erfindungsgemäßen Verfahrens kommen inerte organische Lösungsmittel infrage.

Hierzu gehören insbesondere Ether, wie Ethylenglykoldimethyl- oder -diethylether; Ketone, wie Aceton oder Butanon; Nitrile, wie Acetonitril oder Propionitril; Amide, wie Dimethylformamid, Dimethylacetamid, N-Methylformanilid, N-Methylpyrrolidon oder Hexamethylphosphorsäuretriamid oder Alkohole, wie Methanol, Ethanol oder Propanol.

Die Reaktionstemperaturen können bei der Durchführung des erfindungsgemäßen Verfahrens in einem größeren Bereich variiert werden. Im allgemeinen arbeitet man bei Temperaturen zwischen 0 °C und 100 °C, vorzugsweise bei Temperaturen zwischen 10 °C und 80 °C.

Zur Durchführung des erfindungsgemäßen Verfahrens setzt man pro Mol an substituierten Hydroxypropylamin der Formel (II) äquimolare Mengen an Saccharin ein. Man löst beide Reaktionspartner bei der geeigneten Reaktionstemperatur in einem geeigneten Lösungsmittel und entfernt anschließend das Lösungsmittel durch Destillation im Vakuum. Die Reinigung der so erhältlichen Salze erfolgt mit Hilfe üblicher Reinigungsmethoden, beispielsweise durch Umkristallisieren oder Umfällen. Die Charakterisierung der gelegentlich amorphen Salze erfolgt mit Hilfe spektroskopischer Verfahren (IR; $^{1}$H-NMR).

Die erfindungsgemäßen Wirkstoffe weisen eine starke Wirkung gegen Schädlinge auf und können zur Bekämpfung von unerwünschten Schadorganismen praktisch eingesetzt werden.

Die Wirkstoffe sind für den Gebrauch als Schädlingsbekämpfungsmittel, vor allem als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;

Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei können die erfindungsgemäßen Wirkstoffe mit besonders gutem Erfolg zur Bekämpfung von Getreidekrankheiten, wie beispielsweise gegen den Erreger des echten Gerstenmehltaus (Erysiphe graminis) oder zur Bekämpfung von Reiskrankheiten, wie beispielsweise gegen den Erreger der Reisfleckenkrankheit (Pyricularia oryzae) oder

zur Bekämpfung von Gemüsekrankheiten, wie beispielsweise gegen den Erreger des Gurkenmehltaus (Sphaerotheca fuliginea) eingesetzt werden. Hervorzuheben ist, daß die erfindungsgemäßen Wirkstoffe neben einer guten protektiven Wirksamkeit auch systemische Eigenschaften besitzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermittel und/oder Dispergiermitteln und/oder schaumerzeugenden Mitteln. Im Falle der Benutzung von Wasser als Streckmittel können z. B auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenswasserstoffe, wie Chlorbenzole, Chlorethylene oder Methylenchlorid, aliphatische Kohlenwasserstoffe, wie Cyclohexan oder Pa-

raffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher

Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele

Beispiel 1:

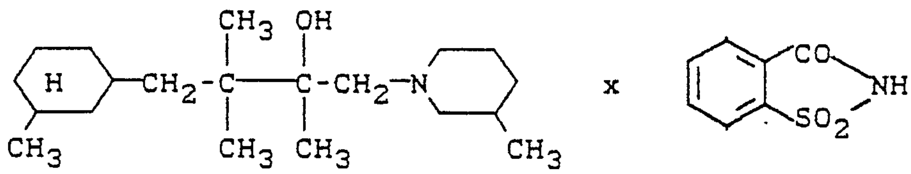

1,2 g (0,0039 Mol) 4-(3-Methylcyclohexyl)-1-(3-methylpiperidin-1-yl)-2,3,3-trimethyl-butan-2-ol in 40 ml Ethanol werden mit 0,7 g (0,00385 Mol) Saccharin versetzt und 10 Minuten bei 50 °C gerührt. Nach Entfernen des Lösungsmittels im Vakuum erhält man 1,9 g (100 % der Theorie) am 4-(3-Methylcyclohexyl)-1-(3-methylpiperidin-1-yl)-2,3,3-trimethyl-butan-2-ol Saccharin-Salz als amorphen Feststoff.

$^1$H-NMR ($CDCl_3$/TMS): $\delta$ = 3,7- 4,0 (m, 2H); 3,55 - 3,1 (m, 3H); 2,8 (m, 1H).

Herstellung der Ausgangsverbindungen der Formel (II)

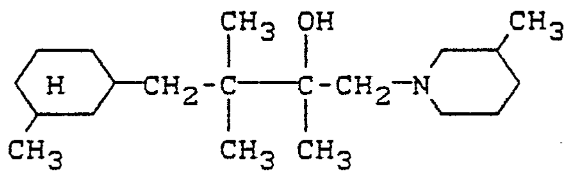

6,8 g (0,022 Mol) 2,3,3-Trimethyl-4-(3-methyl-phenyl)-1-(3-methylpiperidin)-2-butanol werden in 100 ml Isopropanol gelöst und mit 2 g 5%igem Ruthenium-Kohlenstoff bei 130 °C und 200 atm innerhalb von 4 Stunden hydriert. Der Katalysator wird abfiltriert und das Lösungsmittel im Wasserstrahlvakuum abdestilliert.

Man erhält 6,7 g (99 % der Theorie) an 2,3,3-Trimethyl-4-(3-methylcyclohexyl)-1-(3-methylpiperidin-1-yl)-2-butanol mit einem Brechungsindex $n_D^{20}$ : 1,4886.

Die Struktur wird durch NMR-Spektren und die Reinheit durch Gaschromatographie gesichert.

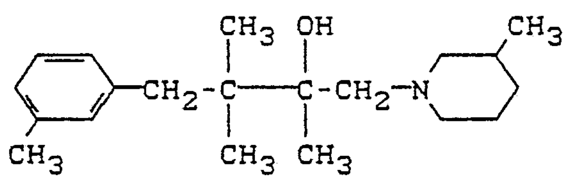

Zu 6,9 g (0,07 Mol) 3-Methylpiperidin in 40 ml n-Butanol und 0,2 ml Eisessig werden 14 g (0,069 Mol) rohes 2,3,3-Trimethyl-4-(3-methylphenyl)-buten-1-oxid in 10 ml n-Butanol zugetropft und 18 Stunden unter Rückfluß erhitzt. Anschließend wird das Lösungsmittel im Wasser-

strahlvakuum abdestilliert. Der Rückstand wird über eine Kieselgelsäule nacheinander mit den Elutionsmitteln Methylenchlorid und Methylenchlorid/Methanol (9:1) chromatographiert.
Man erhält 13,6 g (65 % der Theorie) 2,3,3-Trimethyl-4-(3-methylphenyl)-1-(3-methylpiperidin-1-yl)-2-butanol als Öl mit dem Brechungsindex $n_D^{20}$ : 1.5134.

Die Struktur wird durch NMR-Spektren und die Reinheit durch Gaschromatographie gesichert.

Herstellung der Vorprodukte der Formel (III)

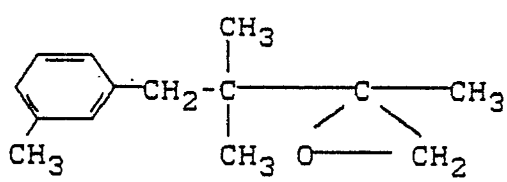

96 g (0,5 Mol) 2,2-Dimethyl-1-(3-methylphenyl)-butan-3-on werden in 630 ml Tetrahydrofuran gelöst. Diese Lösung wird zu einer Suspension von 132,8 g (0,6 Mol) Trimethylsulfoxoniumiodid und 68 g (0,6 Mol) Kalium-tert.-butylat in 160 ml Dimethylsulfoxid, die zuvor 6 Stunden bei 60 °C gerührt wurde, getropft. Die Innentemperatur steigt auf 27 °C an und man rührt 15 Stunden bei 45 °C nach. Anschließend wird das Gemisch auf 2 l Eiswasser gegossen, 4 mal mit jeweils 500 ml Methylenchlorid und 2 mal mit 500 ml Wasser extrahiert. Die organische Phase wird getrocknet und das Lösungsmittel im Vakuum abdestilliert.

Man erhält 97,3 g rohes 2,3,3-Trimethyl-4-(3-methyl-phenyl)-buten-1-oxid.

Herstellung der Vorprodukte der Formel (V)

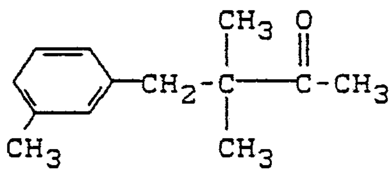

161 g (3 Mol) Kaliumhydroxid werden mit 24,4 g (0,075 Mol) Tetrabutylammoniumbromid in 1,25 l Cyclohexan vorgelegt und unter Rückfluß mit einer Lösung von 258 g (3 Mol) Methylisopropylketon und 210 g (1,511 Mol) 3-Methylbenzylchlorid versetzt. Anschließend wird das Gemisch 30 Stunden unter Rückfluß über einem Wasserabscheider erhitzt, über Kieselgur abgesaugt und im Vakuum destilliert.
Man erhält so 100 g (35 % der Theorie) 2,2-Dimethyl-1-(3-methylphenyl)-butan-3-on vom Siedepunkt 52 °C/0,15 mbar.

In entsprechender Weise und gemäß den allgemeinen Angaben zur Herstellung erhält man die folgenden Saccharin-Salze von substituierten Hydroxypropylaminen der allgemeinen Formel (I):

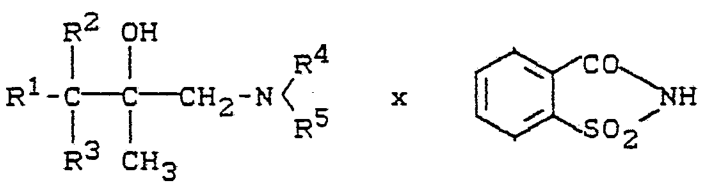

(I)

Tabelle

| Bsp. Nr. | $R^1$ | $R^2$ | $R^3$ | $-N\langle^{R^4}_{R^5}$ | Physikalische Konst. |
|---|---|---|---|---|---|
| 2 | 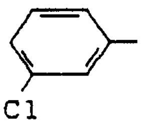 | $CH_3$ | $CH_3$ | 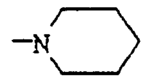 | Fp. 150-151° C |
| 3 | 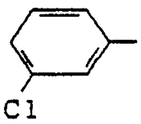 | $CH_3$ | $CH_3$ | 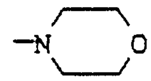 | Fp. 174-175° C |
| 4 | 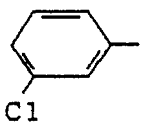 | $CH_3$ | $CH_3$ | 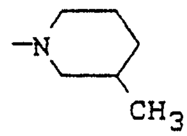 | Fp. 111-113° C |
| 5 | 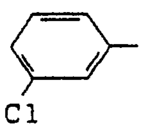 | $CH_3$ | $CH_3$ | 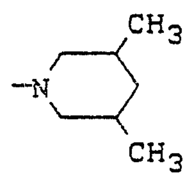 | Fp. 108-110° C |
| 6 | 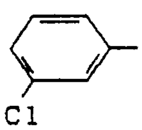 | $CH_3$ | $CH_3$ | 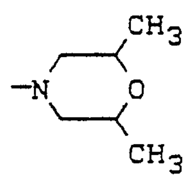 | Fp. 94-99° C |
| 7 | 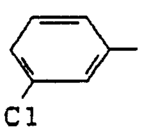 | $CH_3$ | $CH_3$ | 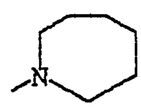 | Fp. 147-150° C |
| 8 | 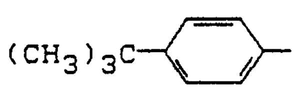 | $CH_3$ | $CH_3$ | 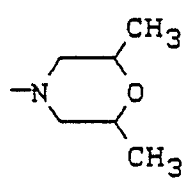 | $^1$H-NMR*): 4,5-4,1 3,7-3,6 3,4-3,3 |

Tabelle - Fortsetzung

| Bsp. Nr. | $R^1$ | $R^2$ | $R^3$ | $-N\langle^{R^4}_{R^5}$ | Physikalische Konst. |
|---|---|---|---|---|---|
| 9 | 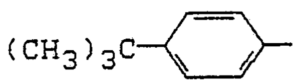 | $CH_3$ | $CH_3$ | 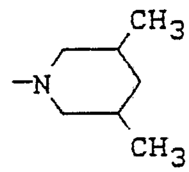 | $^1$H-NMR*): 3,5-2,5 |
| 10 | 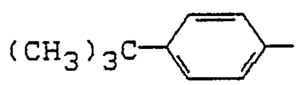 | $CH_3$ | $CH_3$ | 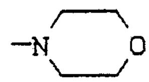 | $^1$HNMR*): 4,2-3,55 3,7-3,2 0,85 |
| 11 | 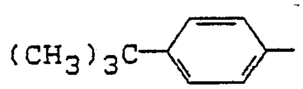 | $CH_3$ | $CH_3$ | 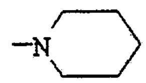 | $^1$H-NMR*): 3,95-3,7 3,35 3,2-3,0 |
| 12 | 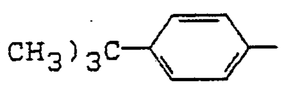 | H | $CH_3$ | 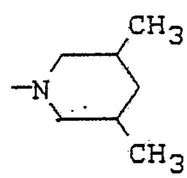 | $^1$H-NMR*): 1,0 |
| 13 | 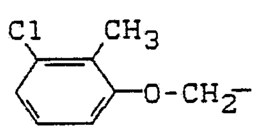 | $CH_3$ | $CH_3$ | 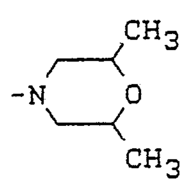 | Fp. 114° - 120° C (Zersetzg.) |
| 14 | 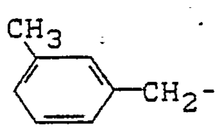 | $CH_3$ | $CH_3$ | 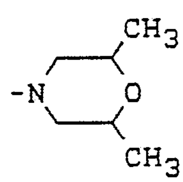 | Fp. 72° - 78° C |
| 15 | 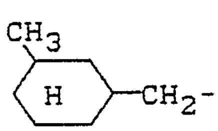 | $CH_3$ | $CH_3$ | 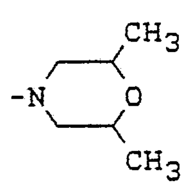 | mp. 75° - 85° C |

*) Die $^1H$-NMR-Spektren wurden in $CDCl_3$ mit Tetramethylsilan (TMS) als innerem Standard aufgenommen. Angegeben ist die chemische Verschiebung als $\delta$-Wert in ppm.

Anwendungsbeispiele

In den folgenden Anwendungsbeispielen wurden die nachstehend aufgeführten Verbindungen als Vergleichssubstanzen eingesetzt:

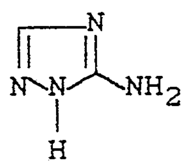 x 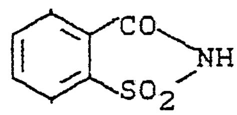 (A)

5-Amino-1,2,4-triazol Saccharin-Salz

und

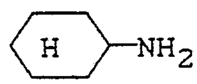 x 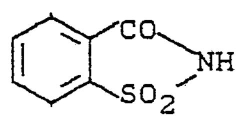 (B)

Cyclohexylamin Saccharin-Salz

(beide bekannt aus EP 158 074)

und

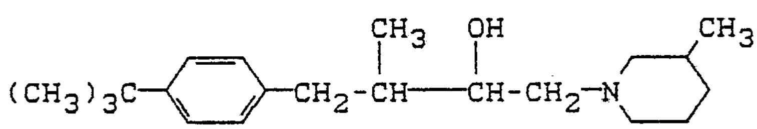 (C)

4-(4-t-Butylphenyl)-3-methyl-1-(3-methylpiperidin-1-yl)-butan-2-ol

(bekannt aus EP 129 321)

Beispiel A

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß den Herstellungsbeispielen: 2, 3, 4, 5, 6, 7, 12.

Beispiel B

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 5,6, 9, 10.

Beispiel C

Pyricularia-Test (Reis) /systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25 °C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 5, 6, 9, 10, 12.

Patentansprüche

1. Saccharin-Salze von substituierten Hydroxypropylaminen der allgemeinen Formel (I),

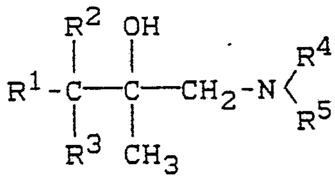

x

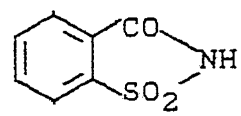

(I)

in welcher

$R^1$ für jeweils gegebenenfalls substituiertes Phenyl, Phenoxy, Phenylthio, Phenylalkyl, Phenoxyalkyl und Phenylthioalkyl oder für jeweils gegebenenfalls substituiertes Cyclohexyl, Cyclohexyloxy, Cyclohexylthio, Cyclohexylalkyl, Cyclohexyloxyalkyl und Cyclohexylthioalkyl steht,.

$R^2$ für Wasserstoff oder Methyl steht,

$R^3$ für Methyl oder Ethyl steht und

$R^4$ und $R^5$ unabhängig voneinander für Alkyl oder Alkenyl stehen oder

$R^4$ und $R^5$ gemeinsam mit dem Stickstoffatom, an das sie gebunden sind, für einen gegebenenfalls substituierten, gesättigten Heterocyclus stehen, der gegebenenfalls weitere Heteroatome enthalten kann.

2. Saccharin-Salze von substituierten Hydroxypropylaminen der Formel (I) gemäß Anspruch 1, in welcher

$R^1$ für jeweils gegebenenfalls einfach bis fünffach, gleich oder verschieden substituiertes Phenyl, Phenoxy, Phenylthio, Phenylalkyl, Phenoxyalkyl und Phenylthioalkyl mit jeweils 1 oder 2 Kohlenstoffatomen im Alkylteil steht, wobei als Phenylsubstituenten jeweils infrage kommen: Halogen, jeweils geradkettiges oder verzweigtes Alkyl, Alkoxy oder Alkylthio mit jeweils 1 bis 5 Kohlenstoffatomen, Halogenalkyl, Halogenalkoxy oder Halogenalkylthio mit jeweils 1 oder 2 Kohlenstoffatomen und 1 bis 5 gleichen oder verschiedenen Halogenatomen; oder für jeweils gegebenenfalls einfach oder mehrfach, gleich oder verschieden substituiertes Cyclohexyl, Cyclohexyloxy, Cyclohexylthio, Cyclohexylalkyl, Cyclohexyloxyalkyl und Cyclohexylthioalkyl mit jeweils 1 oder 2 Kohlenstoffatomen im Alkylteil steht, wobei als Cyclohexylsubstituenten jeweils infrage kommen: jeweils geradkettiges oder verzweigtes Alkyl oder Alkoxy mit jeweils 1 bis 5 Kohlenstoffatomen, Trifluormethyl oder Trifluormethoxy,

$R^2$ für Wasserstoff oder Methyl steht,

$R^3$ für Methyl oder Ethyl steht und

$R^4$ und $R^5$ unabhängig voneinander für geradkettiges oder verzweigtes Alkyl mit 1 bis 8 Kohlenstoffatomen oder für geradkettiges oder verzweigtes Alkenyl mit 3 bis 6 Kohlenstoffatomen stehen oder

$R^4$ und $R^5$ gemeinsam mit dem Stickstoffatom, an welches sie gebunden sind, für einen gegebenenfalls einfach bis zehnfach, gleich oder verschieden substituierten 5- bis 7-gliedrigen, gesättigten Heterocyclus mit 1 oder 2 Heteroatomen stehen, wobei als Substituenten infrage kommen: Methyl, Ethyl und Hydroxymethyl.

3. Saccharin-Salze von substituierten Hydroxylpropylaminen der Formel (I) gemäß Anspruch 1, in welcher

$R^1$ für jeweils gegebenenfalls einfach bis dreifach, gleich oder verschieden substituiertes Phenyl, Phenylmethyl, Phenoxymethyl, Phenylthiomethyl, Phenoxy oder Phenylthio steht, wobei als Phenylsubstituenten jeweils infrage kommen: Fluor, Chlor, Brom, Methyl, Ethyl, n-Propyl, i-Propyl, n-, i-, s- oder t-Butyl, n-Pentyl, 2-Methyl-2-butyl, Methoxy, Ethoxy, n- oder i-Propoxy, t-Butoxy, Trifluormethyl, Trifluormethoxy und Trifluormethylthio; oder für jeweils gegebenenfalls einfach bis dreifach, gleich oder verschieden substituiertes Cyclohexyl, Cyclohexylmethyl, Cyclohexyloxymethyl, Cyclohexylthiomethyl, Cyclohexyloxy oder Cyclohexylthio steht, wobei als Cyclohexylsubstituenten jeweils infrage kommen: Methyl, Ethyl, n- oder i-Propyl, n-, i-, s- oder t-Butyl, n-Pentyl, 2-Methyl-2-butyl, Methoxy, Ethoxy, n- oder i-Propoxy, t-Butoxy, Trifluormethyl und Trifluormethoxy,

$R^2$ für Wasserstoff oder Methyl steht,

$R^3$ für Methyl oder Ethyl steht und

$R^4$ und $R^5$ unabhängig voneinander für Methyl, Ethyl, n-oder i-Propyl, n-, i-, s- oder t-Butyl, n-Pentyl, Allyl, 2-Butenyl oder 3-Methyl-2-butenyl stehen oder

$R^4$ und $R^5$ gemeinsam mit dem Stickstoffatom, an welches sie gebunden sind, für jeweils gegebenenfalls einfach bis dreifach, gleich oder verschieden durch Methyl, Ethyl oder Hydroxymethyl substituiertes 1-Pyrrolidinyl, 1-Piperidinyl, 1-Piperazinyl, 4-Morpholinyl oder 1-Hexahydroazepinyl stehen.

4. Saccharin-Salze von substituierten Hydroxypropylaminen der Formel (I) gemäß Anspruch 1, in welcher

$R^1$ für jeweils gegebenenfalls ein- oder zweifach, gleich oder verschieden durch Chlor, Methyl, t-Butyl und Trifluormethoxy substituiertes Phenyl, Phenoxy, Phenoxymethyl, Phenylthiomethyl, Phenylthio, Cyclohexyloxy, Cyclohexylmethyl oder Cyclohexyloxymethyl steht,

$R^2$ für Wasserstoff oder Methyl steht,

$R^3$ für Methyl steht und

$R^4$ und $R^5$ gemeinsam mit dem Stickstoffatom, an dem sie stehen, für gegebenenfalls ein- bis dreifach durch Methyl substituiertes 1-Piperidinyl, 4-Morpholinyl oder 1-Hexahydroazepinyl stehen.

5. Verfahren zur Herstellung von Saccharin-Salzen von substituierten Hydroxypropylaminen der allgemeinen Formel (I),

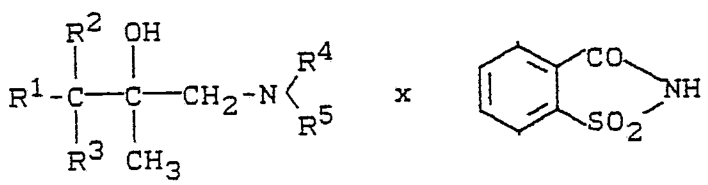

(I)

in welcher

$R^1$ für jeweils gegebenenfalls substituiertes Phenyl, Phenoxy, Phenylthio, Phenylalkyl, Phenoxyalkyl und Phenylthioalkyl oder für jeweils gegebenenfalls substituiertes Cyclohexyl, Cyclohexyloxy, Cyclohexylthio, Cyclohexylalkyl, Cyclohexyloxyalkyl und Cyclohexylthioalkyl steht,

$R^2$ für Wasserstoff oder Methyl steht,

$R^3$ für Methyl oder Ethyl steht und

$R^4$ und $R^5$ unabhängig voneinander für Alkyl oder Alkenyl stehen oder

$R^4$ und $R^5$ gemeinsam mit dem Stickstoffatom, an das sie gebunden sind, für einen gegebenenfalls substituierten, gesättigten Heterocyclus stehen, der gegebenenfalls weitere Heteroatome enthalten kann,

dadurch gekennzeichnet, daß man substituierte Hydroxypropylamine der Formel (II),

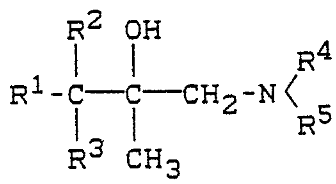

(II)

in welcher

$R^1$, $R^2$, $R^3$, $R^4$ und $R^5$ die oben angegebene Bedeutung haben,

mit Saccharin gegebenenfalls in Gegenwart eines Verdünnungsmittels umsetzt.

6. Schädlingsbekämpfungsmittel, gekennzeichnet durch einen Gehalt am mindestens einem Saccharin-Salz von substituierten Hydroxypropylaminen der Formel (I) gemäß den Ansprüchen 1 und 5.

7. Verwendung von Saccharin-Salzen von substituierten Hydroxypropylaminen der Formel (I) gemäß den Ansprüchen 1 und 5 zur Bekämpfung von Schädlingen.

8. Verfahren zur Bekämpfung von Schädlingen, dadurch gekennzeichnet, daß man Saccharin-Salze von substituierten Hydroxypropylaminen der Formel (I) gemäß den Ansprüchen 1 und 5 auf Schädlinge oder ihren Lebensraum einwirken läßt.

9. Verfahren zur Herstellung von Schädlingsbekämpfungsmitteln, dadurch gekennzeichnet, daß man Saccharin-Salze von substituierten Hydroxypropylaminen der Formel (I) gemäß den Ansprüchen 1 und 5 mit Streckmitteln und/oder oberflächenaktiven Mitteln vermischt.

10. Verwendung von Saccharin-Salzen von substituierten Hydroxypropylaminen der Formel (I) gemäß Anspruch 7 zur Bekämpfung von Pilzen.

Europäisches Patentamt

# EUROPÄISCHER RECHERCHENBERICHT

Nummer der Anmeldung

EP 87 11 6507

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP-A-0 158 074 (BAYEER AG)<br>* Ansprüche; Seite 8, Zeile 18 - Seite 9, Absatz 1 *<br>--- | 1,5-10 | C 07 D 275/06<br>C 07 D 295/08<br>C 07 D 265/30<br>C 07 D 211/14<br>A 01 N 43/80<br>A 01 N 43/40<br>A 01 N 43/84 |
| D,A | EP-A-0 129 321 (IMPERIAL CHEMICAL INDUSTRIES PLC)<br>* Ansprüche 1, 10, 11 *<br>--- | 1,6,8, 10 | |
| A | CHEMICAL ABSTRACTS, Band 79, Nr. 19, 12. November 1973, Seite 86, Zusammenfassungsnr. 112382p, Columbus, Ohio, US; & JP - A - 73 22624 (KUMIAI CHEMICAL INDUSTRY CO., LTD.) 23.03.1973<br>--- | 1,6,7 | |
| A | US-A-3 567 723 (SEKI et al.)<br>* Tabelle 2 *<br>--- | 1,6,7 | |
| A | US-A-2 538 645 (HAMILTON)<br>* Beispiel 3 *<br>----- | 1 | RECHERCHIERTE SACHGEBIETE (Int. Cl.4)<br>C 07 D 275/00 |

Der vorliegende Recherchenbericht wurde für alle Patentansprüche erstellt

| Recherchenort | Abschlußdatum der Recherche | Prüfer |
|---|---|---|
| BERLIN | 05-02-1988 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)